# EUROPÄISCHE PATENTANMELDUNG

(11) **EP 4 542 564 A1**
(43) Veröffentlichungstag der Anmeldung: **23.04.2025**
(21) Anmeldenummer: 23204786.0
(22) Anmeldetag: 20.10.2023
(51) Int. Cl.: G16H 30/20, G06F 3/16, G10L 15/22, G16H 40/63

(54) **MENSCH-MASCHINE-INTERAKTION ZUR STEUERUNG UND/ODER KONFIGURATION EINER MEDIZINTECHNISCHEN ANLAGE**

(71) Anmelder: Siemens Healthineers AG, 91301 Forchheim (DE)
(72) Erfinder: Fernández-Gutiérrez, Fabiola, 90482 Nürnberg (DE); Roser, Philipp, 91052 Erlangen (DE)
(74) Vertreter: Siemens Healthineers Patent Attorneys

(57) **Zusammenfassung**

Zur Mensch-Maschine-Interaktion zur Steuerung und/oder Konfiguration einer medizintechnischen Anlage (1) wird eine Benutzereingabe für ein zentrales computerimplementiertes Sprachmodell, CIS, (11) erfasst, welche eine Durchführung einer medizinischen Untersuchung und/oder Intervention mittels der medizintechnischen Anlage (1) betrifft. Unter Verwendung des zentralen CIS (11) wird abhängig von der Benutzereingabe eine erste Eingabe für ein erstes CIS (12) erzeugt. Unter Verwendung des ersten CIS (12) werden abhängig von der ersten Eingabe erste Anweisungen zur Steuerung und/oder Konfiguration einer ersten medizintechnischen Vorrichtung der medizintechnischen Anlage (1) erzeugt. Abhängig von den ersten Anweisungen wird die erste Vorrichtung (2) wenigstens teilweise automatisch gesteuert und/oder konfiguriert und/oder abhängig von den ersten Anweisungen werden Benutzerinformationen zur Steuerung und/oder Konfiguration der ersten Vorrichtung (2) ausgegeben.

## Beschreibung

Die vorliegende Erfindung betrifft ein computerimplementiertes Verfahren zur Mensch-Maschine-Interaktion zur Steuerung und/oder Konfiguration einer medizintechnischen Anlage. Die Erfindung betrifft ferner eine Datenverarbeitungsvorrichtung zur Durchführung eines solchen computerimplementierten Verfahrens zur Mensch-Maschine-Interaktion, eine medizintechnische Anlage mit einer solchen Datenverarbeitungsvorrichtung, sowie ein Computerprogrammprodukt für die Durchführung eines solchen computerimplementierten Verfahrens.

Medizintechnische Vorrichtungen, beispielsweise Bildgebungsmodalitäten wie Ultraschallbildgebungssysteme, röntgenbasierte Bildgebungssysteme und Magnetresonanztomographiesysteme, C-Bogen-Geräte, verfahrbare beziehungsweise verstellbare Patientenliegen, oder Vorrichtungen zur Durchführung therapeutischer Verfahren, etwa HIFU-Systeme (HIFU: "Hochintensiver fokussierter Ultraschall") oder Histotripsie-Systeme, sind häufig sehr komplex und können eine große Anzahl von Funktionen aufweisen.

Um solche medizintechnischen Vorrichtungen adäquat nutzen zu können, sind häufig nicht nur eine medizinische Grundausbildung und eine Ausbildung spezifisch für die konkrete medizintechnische Vorrichtung erforderlich, sondern in hohem Maße auch Übung und praktische Erfahrung. Zudem ist es gegebenenfalls erforderlich, das jeweilige Wissen über die Arbeitsabläufe und Funktionen stets auf dem neuesten Stand zu halten.

In der Folge kann dies dazu führen, dass medizintechnische Vorrichtungen nicht korrekt bedient werden und/oder deren Funktionalität nicht optimal ausgenutzt wird.

Diese Problematik verschärft sich bei medizintechnischen Anlagen mit mehreren medizintechnischen Vorrichtungen und/oder sonstigen Vorrichtungen. Beispielsweise gibt es bei bildgebungsgestützten Interventionen gegebenenfalls eine Reihe von Vorrichtungen und Vorrichtungskomponenten, mit denen das medizinische Personal interagiert, wie zum Beispiel ein Angiographiesystem, Tischbedienfelder, Anzeigegeräte, EKG-Signalgeräte, Datenbanken zum Abrufen der medizinischen Vorgeschichte des Patienten und so weiter. Diese Vorrichtungen erfordern verschiedenartige Benutzereingaben, beispielsweise per Hand oder per Fuß über ein Pedal. Zugleich können Vorrichtungen miteinander verbunden sein und untereinander Informationen austauschen, was beispielsweise ebenfalls manuell erfolgt. So können beispielsweise Patienteninformationen, die aus einem IT-System des Krankenhauses stammen, an das Angiographiesystem gesendet werden.

Ein weiteres Problem ist, dass die einzelnen Vorrichtungen nicht notwendigerweise über einheitliche Schnittstellen verfügen, über die der Informationsaustausch automatisiert werden könnte.

Computerimplementierte Sprachmodelle, CIS, insbesondere sogenannte große Sprachmodelle, LLMs (englisch: large language models), wie etwa das in der Veröffentlichung "GPT-4 Technical Report" (arXiv:2303.08774v3) beschriebene GPT-4, können als virtuelle Assistenten genutzt werden.

Solche CIS basieren beispielsweise auf Transformer-Netzwerkarchitekturen künstlicher neuronaler Netzwerke, die auf der Grundlage großer Mengen von Trainingsdaten trainiert wurden. Die Trainingsdaten können dabei generisch sein oder auf bestimmte Anwendungsfelder, auch als Domänen bezeichnet, zugeschnitten sein oder es kann einer Kombination beider Ansätze verfolgt werden, wobei beispielsweise ein generisches CIS durch domänenspezifisches weiteres Training für ein konkretes Anwendungsfeld verfeinert wird. Transformer-Netzwerke wurden beispielsweise in der Veröffentlichung A. Vaswani et al.: "Attention Is All You Need" (ArXiv:1706.03762v7) eingeführt.

Eine Herausforderung bei der Nutzung von CIS ist die effiziente und korrekte Formulierung von Benutzereingaben (englisch: "prompting"), sodass das CIS auch tatsächlich eine adäquate und nutzbare Ausgabe liefert.

Es ist eine Aufgabe der vorliegenden Erfindung, ein Verfahren zur Mensch-Maschine-Interaktion zur Steuerung und/oder Konfiguration einer medizintechnischen Anlage anzugeben, bei dem ein Benutzer zuverlässiger unterstützt werden kann, insbesondere auch bei komplexen medizintechnischen Anlagen.

Diese Aufgabe wird durch den Gegenstand des unabhängigen Anspruchs gelöst. Vorteilhafte Weiterentwicklungen und bevorzugte Ausführungsformen sind Gegenstand der abhängigen Ansprüche.

Die Erfindung beruht auf der Idee, ein zentrales CIS vorzusehen, mit dem ein menschlicher Benutzer interagieren kann, und das zentrale CIS zu nutzen, um basierend auf einer entsprechenden Benutzereingabe eine Eingabe (englisch: prompt) für ein weiteres CIS zu erzeugen, welches dann wiederum Anweisungen zur Steuerung und/oder Konfiguration einer dem ersten CIS zugeordneten Vorrichtung der medizintechnischen Anlage erzeugt.

Gemäß einem Aspekt der Erfindung wird ein computerimplementiertes Verfahren zur Mensch-Maschine-Interaktion zur Steuerung und/oder Konfiguration einer medizintechnischen Anlage angegeben. Dabei wird eine Benutzereingabe für ein zentrales computerimplementiertes Sprachmodell, CIS, erfasst, insbesondere mittels einer Datenverarbeitungsvorrichtung über eine Benutzereingabeschnittstelle. Die Benutzereingabe betrifft eine Durchführung einer medizinischen Untersuchung und/oder Intervention mittels der medizintechnischen Anlage, insbesondere unter Verwendung einer ersten Vorrichtung der medizintechnischen Anlage.

Unter Verwendung des zentralen CIS wird abhängig von der Benutzereingabe eine erste Eingabe für ein der ersten Vorrichtung der medizintechnischen Anlage zugeordnetes erstes CIS erzeugt. Unter Verwendung des ersten CIS werden abhängig von der ersten Eingabe erste Anweisungen zur Steuerung und/oder Konfiguration der ersten Vorrichtung erzeugt. Abhängig von den ersten Anweisungen wird die erste Vorrichtung wenigstens teilweise automatisch gesteuert und/oder konfiguriert und/oder es werden abhängig von den ersten Anweisungen Benutzerinformationen zur Steuerung und/oder Konfiguration der ersten Vorrichtung ausgegeben werden, insbesondere mittels einer Benutzerausgabeschnitt-stelle der Datenverarbeitungs-vorrichtung.

Sofern nicht anders angegeben, können alle Schritte des computerimplementierten Verfahrens von der Datenverarbeitungs-vorrichtung durchgeführt werden, welche wenigstens eine Recheneinheit aufweist. Insbesondere ist die wenigstens eine Recheneinheit zur Durchführung der Schritte des computerimplementierten Verfahrens konfiguriert oder angepasst. Hierzu kann die wenigstens eine Recheneinheit beispielsweise ein Computerprogramm speichern, das Befehle beinhaltet, die bei Ausführung durch die wenigstens eine Recheneinheit die wenigstens eine Recheneinheit dazu veranlassen, das computerimplementierte Verfahren auszuführen.

Eine medizintechnische Anlage kann eine technische Anlage zur, insbesondere wenigstens zum Teil automatischen, Durchführung eines diagnostischen, therapeutischen, chirurgischen und/oder bildgebenden Verfahrens sein. Die Durchführung eines diagnostischen, therapeutischen und/oder chirurgischen Verfahrens ist jedoch im Allgemeinen nicht Bestandteil des erfindungsgemäßen computerimplementierten Verfahrens.

Die erste Vorrichtung kann eine medizintechnische Vorrichtung sein, die direkt zur Durchführung des diagnostischen, therapeutischen, chirurgischen und/oder bildgebenden Verfahrens genutzt werden kann, beispielsweise ein röntgenbasiertes Angiographiesystem, insbesondere ein C-Bogen-Gerät, ein Computertomographiesystem, ein Magnetresonanztomographiesystem, ein Ultraschallbildgebungssystem, ein HIFU-System, ein Histotripsiesystem, und so weiter. Die erste Vorrichtung kann aber auch indirekt für das diagnostische, therapeutische, chirurgische und/oder bildgebende Verfahren oder zur Unterstützung desselben genutzt werden. Es kann sich beispielsweise um eine Patientenliege, eine Infrastrukturvorrichtung, wie etwa ein Belüftungssystem oder ein Beleuchtungssystem oder eine Anzeigevorrichtung und so weiter handeln.

Bei einem CIS handelt es sich beispielsweise um ein LLM. Ein CIS kann insbesondere trainiert bereitgestellt werden, also ein an sich bekanntes Sprachmodell sein. Das CIS kann auch untrainiert bereitgestellt werden und anhand von Trainingsdaten in an sich bekannter Weise trainiert werden. Es ist auch möglich, dass das CIS vortrainiert bereitgestellt wird und anhand weiterer Trainingsdaten verfeinert wird, beispielsweise über entsprechend bereitgestellte Schnittstelle zur Programmierung von Anwendungen, API (englisch: application programming interface).

Die Benutzereingabe kann beispielsweise in Textform erfolgen oder auch als Spracheingabe, beispielsweise gefolgt von einer Sprache-zu-Text Umwandlung. Die erste Eingabe für das erste CIS wird insbesondere in Textform erzeugt.

Die Benutzereingabe kann beispielsweise eines oder mehrere Mitglieder des klinischen Teams benennen oder identifizieren und/oder einen Patienten benennen oder identifizieren und/oder eine mittels der medizintechnischen Anlage, insbesondere unter Verwendung der ersten Vorrichtung, durchzuführende Maßnahme, insbesondere eine diagnostische und/oder therapeutische und/oder chirurgische Maßnahme, spezifizieren.

Das zentrale CIS und das erste CIS sind insbesondere voneinander verschieden, also basierend auf jedenfalls zum Teil verschiedenen Trainingsdaten trainiert. Die zugrundeliegende Netzwerkarchitektur kann bei dem zentralen CIS und dem ersten CIS jedoch dieselbe sein. Das erste CIS ist insbesondere ein domänenspezifisches CIS, wobei die Domäne durch die Steuerung und/oder Konfiguration der ersten Vorrichtung gegeben ist. Das zentrale CIS ist insbesondere auf umfassendere Zusammenhänge betreffend die gesamte medizintechnische Anlage ausgelegt beziehungsweise trainiert. Es kann zwar ebenfalls als domänenspezifisches CIS angesehen werden, die Domäne betrifft hierbei jedoch nicht spezifisch die erste Vorrichtung, sondern die Steuerung und/oder Konfiguration der medizintechnischen Anlage, sowie gegebenenfalls klinische Abläufe, die mit der medizintechnischen Anlage realisiert werden können. Im Folgenden umfasst der Begriff eines domänenspezifischen CIS daher nicht das zentrale CIS, sofern nicht ausdrücklich anderweitig angegeben.

Beispielsweise kann das zentrale CIS auf klinische Arbeitsabläufe trainiert sein, die sich aus nationalen und internationalen klinischen Richtlinien, klinikspezifischen Standardarbeitsanweisungen, und/oder historischen Einstellungen.

Das erste CIS als domänenspezifisches CIS ist beispielsweise auf domänenspezifische Informationen betreffend die konkrete erste Vorrichtung oder vergleichbare Vorrichtungen zum selben Zweck trainiert, beispielsweise auf Benutzerhandbüchern, früheren Einstellungen, Verfahren die aus Veröffentlichungen eines Herstellers der ersten Vorrichtung oder basierend auf technischen Inhalten von Marketingmaterial stammen, wissenschaftlichen Fachzeitschriften, und so weiter.

Dass die erste Vorrichtung abhängig von den ersten Anweisungen wenigstens teilweise automatisch gesteuert und/oder konfiguriert wird, kann insbesondere derart verstanden werden, dass mittels der Datenverarbeitungsvorrichtung die entsprechenden Steuerbefehle und/oder Konfigurationen direkt an die erste Vorrichtung oder an Steuermodule der ersten Vorrichtung übermittelt werden, sodass wenigstens ein Teil der Steuerung und/oder Konfiguration der ersten Vorrichtung erfolgt, ohne dass dazu eine weitere Benutzereingabe erforderlich ist.

Die Benutzerinformationen zur Steuerung und/oder Konfiguration der ersten Vorrichtung, die abhängig von den ersten Anweisungen ausgegeben werden, dienen insbesondere dazu, eines oder mehrere Mitglieder des klinischen Teams bei der manuellen oder teilweise manuellen Steuerung und/oder Konfiguration der ersten Vorrichtung anzuleiten.

Das computerimplementierte Verfahren kann insbesondere auf medizintechnische Anlagen erweitert werden, bei denen neben der ersten Vorrichtung auch weitere Vorrichtungen und dementsprechend zugeordnete domänenspezifische CIS vorsieht.

Gemäß dem computerimplementierten Verfahren agiert das zentrale CIS als universelle Schnittstelle zum Benutzer, wohingegen das erste CIS, und gegebenenfalls weitere domänenspezifische CIS, als universelle Schnittstellen zu der ersten Vorrichtung beziehungsweise gegebenenfalls zu weiteren Vorrichtungen der medizintechnischen Anlage. Dadurch wird die Steuerung und/oder Konfiguration der medizintechnischen Anlage insgesamt für das klinische Team entscheidend vereinfacht, und insbesondere kann die Mensch-Maschine-Interaktion mit der medizintechnischen Anlage zentralisiert werden. Dadurch kann jedenfalls zum Teil auf standardisierte Schnittstellen zum Informationsaustausch zwischen verschiedenen Bestandteilen der medizintechnischen Anlage verzichtet werden.

Gemäß zumindest einer Ausführungsform ist die erste Vorrichtung eine medizintechnische Vorrichtung und beinhaltet insbesondere ein Bildgebungssystem.

Aufgrund der hohen Komplexität und Vielzahl verschiedener Konfigurations- und Anwendungsmöglichkeiten medizintechnischer Vorrichtungen, insbesondere von Bildgebungssystemen, wirkt sich hierbei die Erfindung besonders vorteilhaft aus.

Beispielsweise kann die erste Vorrichtung basierend auf den ersten Anweisungen angesteuert werden, eine durch die ersten Anweisungen bestimmte Datenerfassungsaktion oder Datenerfassungssequenz zur Bildgebung einzuleiten oder durchzuführen und/oder eine oder mehrere bewegliche Komponenten der ersten Vorrichtung in eine durch die ersten Anweisungen bestimmte Position und/oder Orientierung zu bewegen oder entlang einer durch die ersten Anweisungen bestimmten Bahn zu führen und so weiter. Beispielsweise können auch Betriebsparameter einer Strahlungsquelle, beispielsweise einer Röntgenstrahlungsquelle, der ersten Vorrichtung und/oder einer Filtervorrichtung, beispielsweise einer Röntgenfiltervorrichtung, der ersten Vorrichtung basierend auf den ersten Anweisungen eingestellt werden, und so weiter.

Gemäß zumindest einer Ausführungsform beinhaltet das Bildgebungssystem ein C-Arm-Röntgenbildgebungssystem, ein Angiographiesystem, ein Computertomographiesystem, ein Magnetresonanztomographiesystem, ein Positronenemissionstomographiesystem und/oder ein Ultraschallbildgebungssystem.

Aufgrund der hohen Komplexität und Vielzahl verschiedener Konfigurations- und Anwendungsmöglichkeiten solcher Bildgebungssysteme wirkt sich hierbei die Erfindung besonders vorteilhaft aus.

Gemäß zumindest einer Ausführungsform wird unter Verwendung des zentralen CIS abhängig von der Benutzereingabe eine zweite Eingabe für ein einer zweiten Vorrichtung der medizintechnischen Anlage zugeordnetes zweites CIS, insbesondere ein zweites domänenspezifisches CIS, erzeugt. Unter Verwendung des zweiten CIS werden abhängig von der zweiten Eingabe zweite Anweisungen zur Steuerung und/oder Konfiguration der zweiten Vorrichtung der medizintechnischen Anlage erzeugt. Abhängig von den zweiten Anweisungen wird die zweite Vorrichtung wenigstens teilweise automatisch gesteuert und/oder konfiguriert und/oder werden abhängig von den zweiten Anweisungen Benutzerinformationen zur Steuerung und/oder Konfiguration der zweiten Vorrichtung ausgegeben, insbesondere mittels der Benutzerausgabeschnittstelle der Datenverarbeitungsvorrichtung.

Die obenstehenden Ausführungen zu dem ersten CIS beziehungsweise der ersten Vorrichtung können analog auf das zweite CIS beziehungsweise die zweite Vorrichtung übertragen werden. Die zweite Vorrichtung ist jedoch im Allgemeinen verschieden von der ersten Vorrichtung und ist beispielsweise keine medizintechnische Vorrichtung im engeren Sinne wie oben beschrieben. Insbesondere sind das erste CIS und das zweite CIS für unterschiedliche Domänen trainiert, also insbesondere basierend auf unterschiedlichen Trainingsdaten.

Das zentrale CIS kann hier also für mindestens zwei verschiedene Vorrichtungen der medizintechnischen Anlage als universelle Benutzerschnittstelle dienen, ohne dass das klinische Team für die den Vorrichtungen zugeordneten domänenspezifischen CIS die zugehörigen Eingaben, auch als Prompts bezeichnet, erstellen muss.

Gemäß zumindest einer Ausführungsform beinhaltet die zweite Vorrichtung eine Beleuchtungsvorrichtung, beispielsweise eine Beleuchtungsvorrichtung zur Beleuchtung eines Raums, insbesondere eines Raums in dem die erste Vorrichtung angeordnet ist.

Dementsprechend können anhand der zweiten Anweisungen die für die durchzuführende Maßnahme und/oder den Patienten und/oder das klinische Team optimale Beleuchtungsbedingungen erzielt werden.

Gemäß zumindest einer Ausführungsform werden unter Verwendung des zentralen CIS abhängig von der Benutzereingabe erste Daten, welche Konfigurationsdaten für die erste Vorrichtung und/oder Patientendaten des Patienten und/oder Bedienerdaten einer Bedienperson der medizintechnischen Anlage, also beispielsweise eines Mitglieds des klinischen Teams, enthalten, aus wenigstens einer Datenbank abgerufen. Die erste Vorrichtung wird abhängig von den ersten Daten, beispielsweise abhängig von den ersten Anweisungen und den ersten Daten, wenigstens teilweise automatisch gesteuert und/oder konfiguriert und/oder die Benutzerinformationen werden abhängig von den ersten Daten, beispielsweise abhängig von den ersten Anweisungen und den ersten Daten, ausgegeben.

Die Konfigurationsdaten können beispielsweise mögliche einstellbare Parameter oder Parameterbereiche oder sonstige Betriebsbereiche einer der Vorrichtungen der medizinischen Anlage und so weiter beinhalten oder betreffen. Die Patientendaten können beispielsweise personenbezogene Daten, wie etwa Größe, Gewicht, Geschlecht, Alter, Gesundheitszustand, Krankheitsverlauf, und so weiter beinhalten oder betreffen. Die Bedienerdaten können beispielsweise die Identität der Bedienperson, personenbezogene Daten, wie etwa die Größe, und/oder bevorzugte Einstellungen der Bedienperson betreffend eine der Vorrichtungen der medizinischen Anlage und so weiter beinhalten oder betreffen.

Dabei kann das zentrale CIS die Konfigurationsdaten, die Patientendaten und/oder die Bedienerdaten direkt aus der wenigstens einen Datenbank abrufen oder indirekt, beispielsweise über ein weiteres domänenspezifisches CIS.

So kann die Durchführung der Untersuchung oder Intervention weiter automatisiert und optimiert werden.

Gemäß zumindest einer Ausführungsform wird unter Verwendung des zentralen CIS abhängig von der Benutzereingabe eine dritte Eingabe für ein drittes CIS erzeugt. Die ersten Daten werden unter Verwendung des dritten CIS abhängig von der dritten Eingabe aus der wenigstens einen Datenbank abgerufen.

Das dritte CIS ruft die ersten Daten dabei insbesondere direkt aus der wenigstens einen Datenbank ab. Basierend auf der dritten Eingabe kann das dritte CIS insbesondere Anweisungen für den Abruf der ersten Daten aus der wenigstens einen Datenbank erzeugen.

So kann die Durchführung der Untersuchung oder Intervention weiter automatisiert und optimiert werden.

Gemäß zumindest einer Ausführungsform wird unter Verwendung des zentralen CIS abhängig von der Benutzereingabe eine vierte Eingabe für ein einer vierten Vorrichtung der medizintechnischen Anlage zugeordnetes viertes CIS, insbesondere ein viertes domänenspezifisches CIS, erzeugt. Unter Verwendung des vierten CIS werden abhängig von der vierten Eingabe vierte Anweisungen zur Steuerung und/oder Konfiguration der vierten Vorrichtung der medizintechnischen Anlage erzeugt. Abhängig von den vierten Anweisungen wird die vierte Vorrichtung wenigstens teilweise automatisch gesteuert und/oder konfiguriert und/oder werden abhängig von den vierten Anweisungen Benutzerinformationen zur Steuerung und/oder Konfiguration der vierten Vorrichtung ausgegeben, insbesondere mittels der Benutzerausgabeschnittstelle der Datenverarbeitungsvorrichtung.

Die obenstehenden Ausführungen zu dem ersten, zweiten und dritten CIS können analog auf das vierte CIS beziehungsweise die vierte Vorrichtung übertragen werden.

Gemäß zumindest einer Ausführungsform beinhaltet die vierte Vorrichtung einen Patiententisch, beispielsweise einen Patiententisch zur Verwendung mit der ersten Vorrichtung.

Beispielsweise ist der Patiententisch gemäß zwei oder mehr Rotationsachsen und/oder Translationsachsen, zumindest teilweise automatisch bewegbar und/oder verstellbar. Die vierten Anweisungen betreffen insbesondere die Bewegung des Patiententisches gemäß den zwei oder mehr Rotationsachsen und/oder Translationsachsen.

Dementsprechend können anhand der zweiten Anweisungen die für die durchzuführende Maßnahme und/oder den Patienten und/oder das klinische Team optimale Einstellungen des Patiententisches, beispielsweise dessen Höhe und/oder Orientierung und/oder Position im Raum und so weiter, erzielt werden.

Gemäß zumindest einer Ausführungsform wird unter Verwendung des zentralen CIS abhängig von der Benutzereingabe eine fünfte Eingabe für ein einer fünften Vorrichtung der medizintechnischen Anlage zugeordnetes fünftes CIS, insbesondere ein fünftes domänenspezifisches CIS, erzeugt. Unter Verwendung des fünften CIS werden abhängig von der fünften Eingabe fünfte Anweisungen zur Steuerung und/oder Konfiguration der fünften Vorrichtung der medizintechnischen Anlage erzeugt. Abhängig von den fünften Anweisungen wird die fünfte Vorrichtung wenigstens teilweise automatisch gesteuert und/oder konfiguriert und/oder werden abhängig von den fünften Anweisungen Benutzerinformationen zur Steuerung und/oder Konfiguration der fünften Vorrichtung ausgegeben, insbesondere mittels der Benutzerausgabeschnittstelle der Datenverarbeitungsvorrichtung.

Die obenstehenden Ausführungen zu dem ersten, zweiten, dritten und vierten CIS können analog auf das fünfte CIS beziehungsweise die fünfte Vorrichtung übertragen werden.

Gemäß zumindest einer Ausführungsform beinhaltet die fünfte Vorrichtung ein Anzeigegerät, insbesondere einen oder mehrere Bildschirme.

Dementsprechend können anhand der fünften Anweisungen die für die durchzuführende Maßnahme und/oder das klinische Team optimale Einstellungen des Anzeigegeräts, beispielsweise dessen Orientierung und/oder Position im Raum, eine Bildschirmaufteilung, eine Auswahl anzuzeigender Daten, und so weiter, erzielt werden.

Gemäß zumindest einer Ausführungsform wird eine weitere Benutzereingabe für das zentrale CIS erfasst wird, welche eine Statusabfrage betreffend Patientenvitalparameter des Patienten und/oder einen Zustand, insbesondere einen aktuellen Zustand, der ersten Vorrichtung beinhaltet. Unter Verwendung des zentralen CIS wird abhängig von der weiteren Benutzereingabe eine weitere erste Eingabe für das erste CIS erzeugt. Unter Verwendung des ersten CIS werden abhängig von der weiteren ersten Eingabe weitere erste Anweisungen zur Abfrage der Patientenvitalparameter und/oder des Zustands der ersten Vorrichtung erzeugt werden. Abhängig von den weiteren ersten Anweisungen werden die Patientenvitalparameter und/oder der Zustand der ersten Vorrichtung erste Vorrichtung wenigstens teilweise automatisch abgefragt.

Die Erläuterungen bezüglich der Benutzereingabe können analog auf die weitere Benutzereingabe übertragen werden. Die Erläuterungen bezüglich der ersten Eingabe und den ersten Anweisungen können analog auf die weitere erste Eingabe beziehungsweise die weiteren ersten Anweisungen übertragen werden.

Die Patientenvitalparameter können beispielsweise einen Blutdruck, eine Pulsfrequenz, eine Atemfrequenz, eine Blutsauerstoffsättigung, EEG-Daten, EKG-Daten und so weiter beinhalten. Die Patientenvitalparameter werden insbesondere von einem oder mehreren Vitalparametersensorvorrichtungen abgerufen. Der Zustand der ersten Vorrichtung kann beispielsweise eine gegenwärtige oder geplante Position und/oder Orientierung einer oder mehrerer beweglicher Komponenten der ersten Vorrichtung, einen oder mehrere gegenwärtig eingestellte oder geplante Betriebsparameter der ersten Vorrichtung und so weiter beinhalten. Der Zustand der ersten Vorrichtung wird insbesondere von der ersten Vorrichtung abgerufen.

So kann die Durchführung der Untersuchung oder Intervention weiter automatisiert und optimiert werden.

Je nachdem, ob die medizinische Anlage die zweite Vorrichtung, die wenigstens eine Datenbank, die vierte Vorrichtung und/oder die fünfte Vorrichtung beinhaltet, kann die Statusabfrage in manchen Ausführungsformen alternativ oder zusätzlich zum Zustand der ersten Vorrichtung einen entsprechenden Zustand der zweiten Vorrichtung, der wenigstens einen Datenbank, der vierten Vorrichtung beziehungsweise der fünften Vorrichtung betreffen. Die Erläuterungen zum Zustand der ersten Vorrichtung können gegebenenfalls analog übertragen werden.

Gemäß zumindest einer Ausführungsform ist die Benutzereingabe eine Texteingabe und/oder ist die weitere Benutzereingabe eine Texteingabe.

Das zentrale CIS kann daher die Benutzereingabe und/oder die weitere Benutzereingabe unmittelbar verarbeiten.

Gemäß zumindest einer Ausführungsform ist die Benutzereingabe eine Spracheinabe. Die Spracheingabe wird, insbesondere mittels eines Sprache-zu-Text-Softwaremoduls der Datenverarbeitungsvorrichtung, in eine Texteingabe umgewandelt, und die erste Eingabe wird abhängig von der Texteingabe erzeugt.

Der Benutzer kann die Benutzereingabe daher durch Sprache tätigen.

Gemäß zumindest einer Ausführungsform ist die weitere Benutzereingabe eine weitere Spracheinabe. Die weitere Spracheingabe wird, insbesondere mittels des Sprache-zu-Text-Softwaremoduls, in eine weitere Texteingabe umgewandelt, und die weitere erste Eingabe wird abhängig von der weiteren Texteingabe erzeugt.

Gemäß zumindest einer Ausführungsform ist oder wird das zentrale CIS basierend auf Trainingsdaten trainiert.

Gemäß zumindest einer Ausführungsform beinhalten die Trainingsdaten vorgegebene klinische Richtlinien und/oder Untersuchungsprotokolle und/oder Interventionsprotokolle für die erste Vorrichtung und/oder wissenschaftliche Veröffentlichungen betreffend mittels der medizintechnischen Anlage durchführbare Untersuchungen und/oder Interventionen.

Auf diese Weise lässt sich das zentrale CIS effektiv für seine Aufgabe als zentrale Benutzerschnittstelle auf übergeordneter Ebene trainieren.

Gemäß zumindest einer Ausführungsform beinhalten die Trainingsdaten historische Benutzereingaben für das zentrale CIS und/oder historische Konfigurationsdaten für die medizintechnische Anlage.

Auf diese Weise lässt sich das zentrale CIS effektiv für seine Aufgabe als zentrale Benutzerschnittstelle auf übergeordneter Ebene trainieren.

Gemäß zumindest einer Ausführungsform ist oder wird das erste CIS basierend auf weiteren Trainingsdaten trainiert.

Gemäß zumindest einer Ausführungsform beinhalten die weiteren Trainingsdaten wenigstens ein Benutzerhandbuch und/oder wenigstens eine Bedienungsanleitung und/oder wenigstens eine Beschreibung für die erste Vorrichtung.

Auf diese Weise lässt sich das erste CIS effektiv domänenspezifisch trainieren.

Gemäß zumindest einer Ausführungsform beinhalten die weiteren Trainingsdaten vorgegebene klinische Richtlinien und/oder Untersuchungsprotokolle und/oder Interventionsprotokolle für die erste Vorrichtung und/oder wissenschaftliche Veröffentlichungen betreffend mittels der ersten Vorrichtung durchführbare Untersuchungen und/oder Interventionen.

Auf diese Weise lässt sich das erste CIS effektiv domänenspezifisch trainieren.

Gemäß zumindest einer Ausführungsform beinhalten die weiteren Trainingsdaten historische Konfigurationsdaten und/oder Steuerungsdaten für die erste Vorrichtung.

Auf diese Weise lässt sich das erste CIS effektiv domänenspezifisch trainieren.

Gemäß zumindest einer Ausführungsform ist oder wird das zweite CIS und/oder das dritte CIS und/oder das vierte CIS und/oder das fünfte CIS basierend auf jeweiligen Trainingsdaten trainiert. Die Erläuterungen und Ausführungsformen bezüglich der weiteren Trainingsdaten zum Trainieren des ersten CIS lassen sich analog auf die jeweiligen Trainingsdaten zum Trainieren des zweiten CIS, des dritte CIS, des vierte CIS beziehungsweise des fünften CIS übertragen.

Gemäß zumindest einer Ausführungsform beinhaltet das zentrale CIS ein trainiertes künstliches neuronales Netzwerk, welches als Transformer-Netzwerk ausgestaltet ist und/oder das erste CIS beinhaltet ein weiteres trainiertes künstliches neuronales Netzwerk, welches als Transformer-Netzwerk ausgestaltet ist.

Solche neuronalen Netzwerke, wie etwa das eingangs erwähnte GPT-4, haben sich als besonders leistungsfähig erwiesen.

Gemäß zumindest einer Ausführungsform beinhaltet das zweite CIS und/oder das dritte CIS und/oder das vierte CIS und/oder das fünfte CIS jeweils ein entsprechendes trainiertes künstliches neuronales Netzwerk, welches als Transformer-Netzwerk ausgestaltet ist.

Für Anwendungsfälle oder Anwendungssituationen, die sich bei einem erfindungsgemäßen computerimplementierten Verfahren ergeben können und die hierin nicht explizit beschrieben sind, kann vorgesehen sein, dass gemäß dem Verfahren eine Fehlermeldung und/oder eine Aufforderung zur Eingabe einer Nutzerrückmeldung ausgegeben und/oder eine Standard-einstellung und/oder ein vorbestimmter Initialzustand eingestellt wird.

Gemäß einem weiteren Aspekt der Erfindung wird eine Datenverarbeitungsvorrichtung, aufweisend wenigstens eine Recheneinheit, angegeben. Die wenigstens eine Recheneinheit ist dazu eingerichtet, ein erfindungsgemäßes computerimplementiertes Verfahren durchzuführen.

Unter einer Recheneinheit kann insbesondere ein Datenverarbeitungsgerät verstanden werden, das einen Verarbeitungsschaltkreis enthält. Die Recheneinheit kann also insbesondere Daten zur Durchführung von Rechenoperationen verarbeiten. Darunter fallen gegebenenfalls auch Operationen, um indizierte Zugriffe auf eine Datenstruktur, beispielsweise eine Umsetzungstabelle, LUT (englisch: "look-up table"), durchzuführen.

Die Recheneinheit kann insbesondere einen oder mehrere Computer, einen oder mehrere Mikrocontroller und/oder einen oder mehrere integrierte Schaltkreise enthalten, beispielsweise eine oder mehrere anwendungsspezifische integrierte Schaltungen, ASIC (englisch: "application-specific integrated circuit"), eines oder mehrere feldprogrammierbare Gate-Arrays, FPGA, und/oder eines oder mehrere Einchipsysteme, SoC (englisch: "system on a chip"). Die Recheneinheit kann auch einen oder mehrere Prozessoren, beispielsweise einen oder mehrere Mikroprozessoren, eine oder mehrere zentrale Prozessoreinheiten, CPU (englisch: "central processing unit"), eine oder mehrere Grafikprozessoreinheiten, GPU (englisch: "graphics processing unit") und/oder einen oder mehrere Signalprozessoren, insbesondere einen oder mehrere Digitalsignalprozessoren, DSP, enthalten. Die Recheneinheit kann auch einen physischen oder einen virtuellen Verbund von Computern oder sonstigen der genannten Einheiten beinhalten.

In verschiedenen Ausführungsbeispielen beinhaltet die Recheneinheit eine oder mehrere Hardware- und/oder Softwareschnittstellen und/oder eine oder mehrere Speichereinheiten.

Eine Speichereinheit kann als flüchtiger Datenspeicher, beispielsweise als dynamischer Speicher mit wahlfreiem Zugriff, DRAM (englisch: "dynamic random access memory") oder statischer Speicher mit wahlfreiem Zugriff, SRAM (englisch: "static random access memory"), oder als nicht-flüchtiger Datenspeicher, beispielsweise als Festwertspeicher, ROM (englisch: "read-only memory"), als programmierbarer Festwertspeicher, PROM (englisch: "programmable read-only memory"), als löschbarer programmierbarer Festwertspeicher, EPROM (englisch: "erasable programmable read-only memory"), als elektrisch löschbarer programmierbarer Festwertspeicher, EEPROM (englisch: "electrically erasable programmable read-only memory"), als Flash-Speicher oder Flash-EEPROM, als ferroelektrischer Speicher mit wahlfreiem Zugriff, FRAM (englisch: "ferroelectric random access memory"), als magnetoresistiver Speicher mit wahlfreiem Zugriff, MRAM (englisch: "magnetoresistive random access memory") oder als Phasenänderungsspeicher mit wahlfreiem Zugriff, PCR_AM (englisch: "phase-change random access memory"), ausgestaltet sein.

Gemäß zumindest einer Ausführungsform der Datenverarbeitungsvorrichtung speichert die wenigstens eine Recheneinheit das zentrale CIS und das erste CIS.

Gemäß zumindest einer Ausführungsform der Datenverarbeitungsvorrichtung speichert die wenigstens eine Recheneinheit das zweite CIS und/oder das dritte CIS und/oder das vierte CIS und/oder das fünfte CIS.

Gemäß einem weiteren Aspekt der Erfindung wird eine medizintechnische Anlage angegeben, welche die Datenverarbeitungsvorrichtung sowie die erste Vorrichtung aufweist.

Gemäß zumindest einer Ausführungsform der medizintechnischen Anlage beinhaltet die medizintechnische Anlage die zweite Vorrichtung und/oder die wenigstens eine Datenbank und/oder die vierte Vorrichtung und/oder die fünfte Vorrichtung.

Weitere Ausführungsformen der erfindungsgemäßen medizintechnischen Anlage folgen unmittelbar aus den verschiedenen Ausgestaltungen des erfindungsgemäßen computerimplementierten Verfahrens und umgekehrt. Insbesondere lassen sich einzelne Merkmale und entsprechende Erläuterungen sowie Vorteile bezüglich der verschiedenen Ausführungsformen zu dem erfindungsgemäßen computerimplementierten Verfahren analog auf entsprechende Ausführungsformen der erfindungsgemäßen medizintechnischen Anlage übertragen.

Gemäß einem weiteren Aspekt der Erfindung wird ein Computerprogramm mit Befehlen angegeben. Bei Ausführung der Befehle durch eine Datenverarbeitungsvorrichtung, insbesondere eine erfindungsgemäße Datenverarbeitungs-vorrichtung, veranlassen die Befehle die Datenverarbeitungs-vorrichtung dazu, ein erfindungsgemäßes computer-implementiertes Verfahren durchzuführen.

Die Befehle können beispielsweise als Programmcode vorliegen. Der Programmcode kann beispielsweise als Binärcode oder Assembler und/oder als Quellcode einer Programmiersprache, zum Beispiel C, und/oder als Programmskript, zum Beispiel Python, bereitgestellt sein.

Gemäß einem weiteren Aspekt der Erfindung wird ein computerlesbares Speichermedium angegeben, das ein erfindungsgemäßes Computerprogramm speichert.

Das Computerprogramm und das computerlesbare Speichermedium sind jeweils Computerprogrammprodukte mit den Befehlen.

Weitere Merkmale und Merkmalskombinationen der Erfindung ergeben sich aus den Figuren und deren Beschreibung sowie aus den Ansprüchen. Insbesondere müssen weitere Ausführungsformen der Erfindung nicht unbedingt alle Merkmale eines der Ansprüche enthalten. Weitere Ausführungsformen der Erfindungen können Merkmale oder Merkmalskombinationen aufweisen, die nicht in den Ansprüchen genannt sind.

Die Erfindung wird im Folgenden anhand konkreter Ausführungsbeispiele und zugehöriger schematischer Zeichnungen näher erläutert. In den Figuren können gleiche oder funktionsgleiche Elemente mit denselben Bezugszeichen versehen sein. Die Beschreibung gleicher oder funktionsgleicher Elemente wird gegebenenfalls nicht notwendigerweise bezüglich verschiedener Figuren wiederholt.

In den Figuren zeigen
- FIG 1: eine schematische Darstellung einer beispielhaften Ausführungsform einer erfindungsgemäßen medizintechnischen Anlage;
- FIG 2: ein schematisches Ablaufdiagramm einer beispielhaften Ausführungsform eines erfindungsgemäßen computerimplementierten Verfahrens zur MenschMaschine-Interaktion zur Steuerung und/oder Konfiguration einer medizintechnischen Anlage; und
- FIG 3: eine schematische Darstellung einer weiteren beispielhaften Ausführungsform einer erfindungsgemäßen medizintechnischen Anlage.

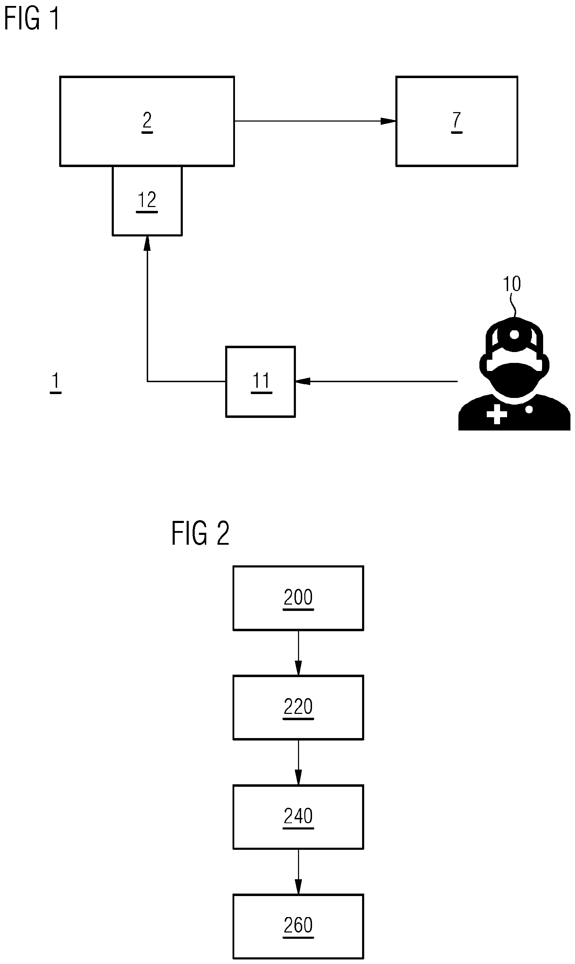

In FIG 1 ist schematisch eine beispielhafte Ausführungsform einer erfindungsgemäßen medizintechnischen Anlage 1 dargestellt.

Die medizintechnische Anlage 1 weist eine erfindungsgemäße Datenverarbeitungsvorrichtung mit wenigstens einer Recheneinheit auf, die dazu angepasst ist, ein erfindungsgemäßes computerimplementiertes Verfahren zur Mensch-Maschine-Interaktion zur Steuerung und/oder Konfiguration der medizintechnischen Anlage 1 durchzuführen. Die medizintechnische Anlage 1 weist außerdem eine erste Vorrichtung 2 auf, beispielsweise ein Bildgebungssystem. Die wenigstens einer Recheneinheit speichert ein zentrales CIS 11 und ein der ersten Vorrichtung 2 zugeordnetes erstes CIS 12.

FIG 2 zeigt ein schematisches Ablaufdiagramm einer beispielhaften Ausführungsform eines erfindungsgemäßen computerimplementierten Verfahrens zur Mensch-Maschine-Interaktion zur Steuerung und/oder Konfiguration der medizintechnischen Anlage 1 der FIG 1.

In Schritt 200 erfasst die wenigstens einer Recheneinheit über eine Benutzereingabeschnittstelle, beispielsweise als Texteingabe oder als Spracheingabe, eine Benutzereingabe eines Benutzers 10 für das zentrale CIS 11. Die Benutzereingabe betrifft eine Durchführung einer medizinischen Untersuchung und/oder Intervention mittels der medizintechnischen Anlage 1, insbesondere unter Verwendung der ersten Vorrichtung 2.

In Schritt 220 wird unter Verwendung des zentralen CIS 11 abhängig von der Benutzereingabe, gegebenenfalls nach Umwandlung der Spracheingabe in Text, eine erste Eingabe für das der ersten Vorrichtung 2 zugeordnete erste CIS 12 erzeugt. In Schritt 240 werden unter Verwendung des ersten CIS 12 abhängig von der ersten Eingabe erste Anweisungen zur Steuerung und/oder Konfiguration der ersten Vorrichtung 2 erzeugt.

In Schritt 260 wird abhängig von den ersten Anweisungen die erste Vorrichtung 2 wenigstens teilweise automatisch gesteuert und/oder konfiguriert. Alternativ oder zusätzlich werden abhängig von den ersten Anweisungen Benutzerinformationen zur Steuerung und/oder Konfiguration der ersten Vorrichtung 2 ausgegeben, insbesondere über eine Benutzerausgabevorrichtung 7, beispielsweise ein Display, der medizintechnischen Anlage 1.

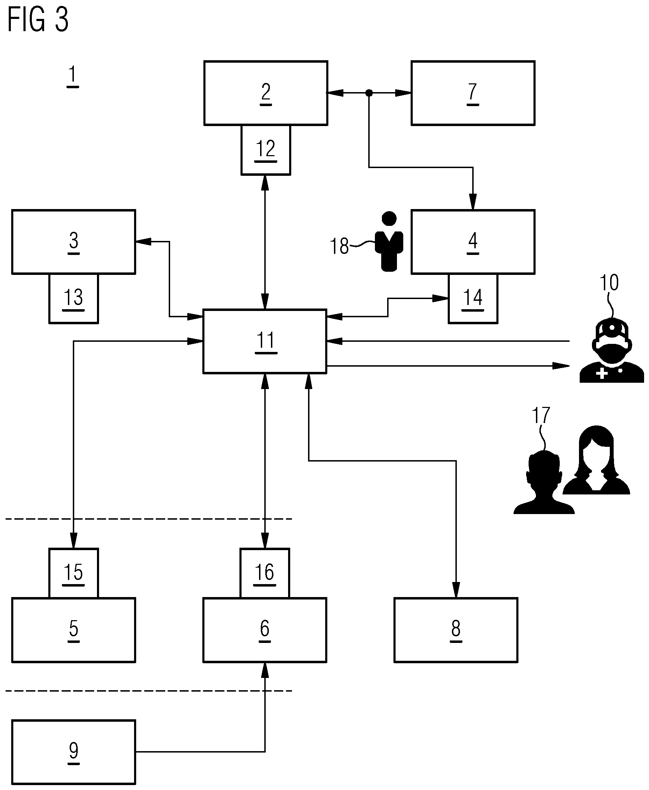

In FIG 3 ist schematisch eine weitere beispielhafte Ausführungsform einer erfindungsgemäßen medizintechnischen Anlage 1 dargestellt, die auf der in FIG 1 dargestellten medizintechnischen Anlage 1 basiert.

Die medizintechnische Anlage 1 gemäß FIG 3 weist außer der ersten Vorrichtung 2 eine zweite Vorrichtung 3, beispielsweise eine Raumbeleuchtungsvorrichtung, und/oder eine dritte Vorrichtung 4, beispielsweise einen Patiententisch, auf. Die medizintechnische Anlage 1 weist dementsprechend auch ein der zweiten Vorrichtung 3 zugeordnetes zweites CIS 13 und/oder ein der dritten Vorrichtung 4 zugeordnetes viertes CIS 14 auf.

Unter Verwendung des zentralen CIS 11 kann abhängig von der Benutzereingabe eine zweite Eingabe das zweite CIS 13 erzeugt werden und/oder eine dritte Eingabe für das dritte CIS 14. Unter Verwendung des zweiten CIS 13 können abhängig von der zweiten Eingabe zweite Anweisungen zur Steuerung und/oder Konfiguration der zweiten Vorrichtung 3 erzeugt werden beziehungsweise abhängig von der dritten Eingabe dritte Anweisungen zur Steuerung und/oder Konfiguration der dritten Vorrichtung 4. Abhängig von den zweiten Anweisungen wird die zweite Vorrichtung 3 beispielsweise wenigstens teilweise automatisch gesteuert und/oder konfiguriert, also insbesondere die Raumbeleuchtung eingestellt. Alternativ oder zusätzlich wird abhängig von den dritten Anweisungen die dritte Vorrichtung 4 beispielsweise wenigstens teilweise automatisch gesteuert und/oder konfiguriert, also insbesondere die Position und/oder Orientierung des Patiententischs eingestellt und/oder dessen Bewegung geplant.

Beispielsweise kann die medizintechnische Anlage 1 eine Datenbank 5 betreffend Konfigurationen der ersten Vorrichtung 2 aufweisen sowie optional ein der Datenbank 5 zugeordnetes viertes CIS 15.

Unter Verwendung des zentralen CIS 11 können abhängig von der Benutzereingabe beispielsweise Konfigurationsdaten für die erste Vorrichtung 2 aus der Datenbank 5 abgerufen werden. Dazu kann das zentrale CIS 11 direkt auf die Datenbank 5 zugreifen oder eine vierte Eingabe für das vierte CIS 15 erzeugen, welches die Konfigurationsdaten abhängig von der vierten Eingabe aus der Datenbank 5 abruft.

Beispielsweise kann die medizintechnische Anlage 1 eine Schnittstelle 6 zu einer externen Datenbank 9 aufweisen, sowie optional ein der Schnittstelle 6 zugeordnetes fünftes CIS 16.

Unter Verwendung des zentralen CIS 11 können abhängig von der Benutzereingabe beispielsweise Patientendaten eines Patienten 18, an dem die Untersuchung oder Intervention durchgeführt werden soll, aus der Datenbank 9 abgerufen werden. Dazu kann das zentrale CIS 11 direkt auf die Schnittstelle 6 zum Abrufen der Datenbank 9 zugreifen oder eine fünfte Eingabe für das fünfte CIS 16 erzeugen, welches die Patientendaten abhängig von der fünften Eingabe über die Schnittstelle 6 aus der Datenbank 9 abruft.

Beispielsweise kann die medizintechnische Anlage 1 eine Datenbank 8 aufweisen. Unter Verwendung des zentralen CIS 11 können abhängig von der Benutzereingabe beispielsweise Bedienerdaten einer Bedienperson 10, 17 der medizintechnischen Anlage 1 aus der Datenbank 8 abgerufen werden. Das zentrale CIS kann dazu beispielsweise direkt auf die Datenbank 8 zugreifen.

Die Bedienerdaten und/oder die Patientendaten und/oder die Konfigurationsdaten können dann beispielsweise verwendet werden, um die erste Vorrichtung 2 wenigstens teilweise automatisch zu steuern und/oder zu konfigurieren.

In einer beispielhaften Ausführungsform kann die erste Vorrichtung 2 ein röntgenbasiertes Angiographiesystem sein, die zweite Vorrichtung 3 die Raumbeleuchtungsvorrichtung, und die dritte Vorrichtung 4 der Patiententisch.

In der Benutzereingabe kann der Benutzer 10, beispielsweise ein leitender Arzt, sich selbst und die übrigen Mitglieder 17 des klinischen Teams identifizieren, ebenso wie den Patienten 18 und die durchzuführende Untersuchung oder Intervention.

Das zentrale CIS 11 kann basierend auf der Benutzereingabe beispielsweise aus der Datenbank 8 bevorzugte Einstellungen betreffend die Raumbeleuchtungsvorrichtung, den Patiententisch und/oder die Benutzerausgabevorrichtung 7 abrufen.

Das zentrale CIS 11 kann basierend auf der Benutzereingabe die fünfte Eingabe für das fünfte CIS 16 erzeugen und dieses kann basierend darauf die Patienteninformationen aus der Datenbank 9 abrufen, um die Einstellung des Patiententisches und des Angiographiesystems einzuleiten.

Das zentrale CIS 11 kann basierend auf der Benutzereingabe die zweite Eingabe für das zweite CIS 13 erzeugen und dieses kann basierend darauf Anweisungen für die Raumbeleuchtungsvorrichtung erzeugen, um die Raumbeleuchtung entsprechend einzustellen.

Das zentrale CIS 11 kann basierend auf der Benutzereingabe die dritte Eingabe für das dritte CIS 14 erzeugen und dieses kann basierend darauf Anweisungen für den Patiententisch erzeugen, um den Patiententisch entsprechend einzustellen.

Wie insbesondere unter Bezugnahme auf die Figuren beschrieben, ermöglicht es die Erfindung, ein Verfahren zur Mensch-Maschine-Interaktion zur Steuerung und/oder Konfiguration einer medizintechnischen Anlage zu realisieren, bei dem ein Benutzer zuverlässiger unterstützt werden kann.

Das zentrale CIS versteht in verschiedenen Ausführungsformen Präferenzen und/oder personenspezifische Einstellungen von Mitgliedern eines medizinischen Teams, beispielsweise basierend auf der Historie durchgeführter Untersuchungen und/oder Interventionen mittels der medizintechnischen Anlage oder einer vergleichbaren medizintechnischen Anlage.

In verschiedenen Ausführungsformen enthält die gespeicherte Historie auch Verfahrens- und/oder Patientenmerkmale. In verschiedenen Ausführungsformen kann das zentrale CIS dem klinischen Team per Sprachsteuerung kommunizieren und/oder mit weiteren CIS, insbesondere dem ersten und/oder dem zweiten CIS, textbasiert.

In verschiedenen Ausführungsformen kann das zentrale CIS entscheiden, welches der domänenspezifischen CIS eingesetzt werden sollte oder Informationen bereitstellen sollte, um die Untersuchung oder Intervention zu beginnen oder fortzusetzen. Enthält die Benutzereingabe beispielsweise nicht genügend Informationen um den nächsten anstehenden Schritt durchzuführen, kann das zentrale CIS die entsprechenden Informationen von dem Benutzer anfordern, zum Beispiel auch als Sprachausgabe.

In verschiedenen Ausführungsformen kann das zentrale CIS auch als Informationsvermittler zwischen den domänenspezifischen CIS fungieren. Es kann beispielsweise überwachen, welche Informationen zwischen den domänenspezifischen CIS ausgetauscht werden und beispielsweise entsprechend reagieren, wenn der Informationsaustausch zwischen den domänenspezifischen CIS unvollständige oder falsche Informationen enthält.

In verschiedenen Ausführungsformen sind mehrere domänenspezifische CIS vorgesehen, die für die jeweilige Domäne trainiert wurden. Diese CIS könne basierend auf geräte- und domänenspezifischen Informationen trainiert sein. Beispielsweise könnte ein einem Angiographiesystem zugeordnetes CIS basierend auf Informationen trainiert werden, die entsprechende Benutzerhandbücher, frühere Einstellungen, die in Krankenhäusern verwendet wurden, und bewährte Verfahren die aus vertrauenswürdigen Quellen stammen, zum Beispiel vom Hersteller des Angiographiesystem oder aus renommierten wissenschaftlichen Fachzeitschriften. Die domänenspezifischen CIS die an der entsprechenden Vorrichtung steuerbaren Funktionen überwachen und steuern, insbesondere im Austausch mit dem zentralen CIS. Falls eine Anfrage des zentralen CIS nicht ausreicht, um die entsprechende Aufgabe zu erfüllen, fordert das jeweilige domänenspezifische CIS auch zusätzliche Informationen bei dem zentralen CIS anfordern.

In verschiedenen Ausführungsformen kann sich jedes domänenspezifische CIS bei dem zentralen CIS registrieren und beispielsweise spezifische Informationen über seine Fähigkeiten, seinen vorgesehenen oder definierten Zweck und so weiter bereitstellen. Auf diese Weise kann das zentrale CIS feststellen, welche Eingabe an welches domänenspezifische CIS übermittelt werden soll.

In verschiedenen Ausführungsformen kann ein Benutzer kann auch interdisziplinäre Anfragen stellen, zum Beispiel in welchem Bereich bestimmte Patientenvitalparameter liegen, und so weiter. Das zentrale CIS kann dann bei den entsprechenden domänenspezifischen CIS anfragen und dem Benutzer beispielsweise eine entsprechenden Antwort zusammenstellen.

Unabhängig vom grammatikalischen Geschlecht eines bestimmten Begriffes sind Personen mit männlicher, weiblicher oder anderer Geschlechteridentität mit umfasst. Dies beinhaltet insbesondere aber nicht nur die Begriffe "Patient", "Benutzer", und "Arzt".

## Patentansprüche

1. Computerimplementiertes Verfahren zur Mensch-Maschine-Interaktion zur Steuerung und/oder Konfiguration einer medizintechnischen Anlage (1), wobei
- eine Benutzereingabe für ein zentrales computerimplementiertes Sprachmodell, CIS, (11) erfasst wird, welche eine Durchführung einer medizinischen Untersuchung und/oder Intervention mittels der medizintechnischen Anlage (1) betrifft;
- unter Verwendung des zentralen CIS (11) abhängig von der Benutzereingabe eine erste Eingabe für ein einer ersten Vorrichtung (2) der medizintechnischen Anlage (1) zugeordnetes erstes CIS (12) erzeugt wird;
- unter Verwendung des ersten CIS (12) abhängig von der ersten Eingabe erste Anweisungen zur Steuerung und/oder Konfiguration der ersten Vorrichtung (2) erzeugt werden; und
- abhängig von den ersten Anweisungen die erste Vorrichtung (2) wenigstens teilweise automatisch gesteuert und/oder konfiguriert wird und/oder abhängig von den ersten Anweisungen Benutzerinformationen zur Steuerung und/oder Konfiguration der ersten Vorrichtung (2) ausgegeben werden.

2. Computerimplementiertes Verfahren nach Anspruch 1, wobei die erste Vorrichtung (2) ein Bildgebungssystem beinhaltet.

3. Computerimplementiertes Verfahren nach Anspruch 2, wobei das Bildgebungssystem ein C-Arm-Röntgenbildgebungssystem, ein Angiographiesystem, ein Computertomographiesystem, ein Magnetresonanztomographiesystem, ein Positronenemissionstomographiesystem und/oder ein Ultraschallbildgebungssystem beinhaltet.

4. Computerimplementiertes Verfahren nach einem der vorhergehenden Ansprüche, wobei
- unter Verwendung des zentralen CIS (11) abhängig von der Benutzereingabe eine zweite Eingabe für ein einer zweiten Vorrichtung (3, 4, 5) der medizintechnischen Anlage (1) zugeordnetes zweites CIS (13, 14) erzeugt wird;
- unter Verwendung des zweiten CIS (13, 14) abhängig von der zweiten Eingabe zweite Anweisungen zur Steuerung und/oder Konfiguration der zweiten Vorrichtung (3, 4, 5) erzeugt werden; und
- abhängig von den zweiten Anweisungen die zweite Vorrichtung (3, 4, 5) wenigstens teilweise automatisch gesteuert und/oder konfiguriert wird und/oder abhängig von den zweiten Anweisungen Benutzerinformationen zur Steuerung und/oder Konfiguration der zweiten Vorrichtung (3, 4, 5) ausgegeben werden.

5. Computerimplementiertes Verfahren nach Anspruch 4, wobei die zweite Vorrichtung (3, 4, 5) eine Beleuchtungsvorrichtung (3) und/oder einen Patiententisch (4) und/oder ein Anzeigegerät (5) beinhaltet.

6. Computerimplementiertes Verfahren nach einem der vorhergehenden Ansprüche, wobei
- unter Verwendung des zentralen CIS (11) abhängig von der Benutzereingabe erste Daten, welche Konfigurationsdaten für die erste Vorrichtung (2) und/oder Patientendaten und/oder Bedienerdaten einer Bedienperson (10, 17) der medizintechnischen Anlage (1) enthalten, aus wenigstens einer Datenbank (5, 8, 9) abgerufen werden; und
- die erste Vorrichtung (2) abhängig von den ersten Daten wenigstens teilweise automatisch gesteuert und/oder konfiguriert wird und/oder die Benutzerinformationen abhängig von den ersten Daten ausgegeben werden.

7. Computerimplementiertes Verfahren nach Anspruch 6, wobei
- unter Verwendung des zentralen CIS (11) abhängig von der Benutzereingabe eine dritte Eingabe für ein drittes CIS (15, 16) erzeugt wird; und
- die ersten Daten unter Verwendung des dritten CIS (15, 16) abhängig von der dritten Eingabe aus der wenigstens einen Datenbank abgerufen werden.

8. Computerimplementiertes Verfahren nach einem der vorhergehenden Ansprüche, wobei
- eine weitere Benutzereingabe für das zentrale CIS (11) erfasst wird, welche eine Statusabfrage betreffend Patientenvitalparameter und/oder einen Zustand der ersten Vorrichtung (2) beinhaltet;
- unter Verwendung des zentralen CIS (11) abhängig von der weiteren Benutzereingabe eine weitere erste Eingabe für das erste CIS (12) erzeugt wird;
- unter Verwendung des ersten CIS (12) abhängig von der weiteren ersten Eingabe weitere erste Anweisungen zur Abfrage der Patientenvitalparameter und/oder des Zustands der ersten Vorrichtung (2) erzeugt werden; und
- abhängig von den weiteren ersten Anweisungen die Patientenvitalparameter und/oder der Zustand der ersten Vorrichtung (2) erste Vorrichtung (2) wenigstens teilweise automatisch abgefragt werden.

9. Computerimplementiertes Verfahren nach einem der vorhergehenden Ansprüche, wobei
- die Benutzereingabe eine Texteingabe ist; oder
- die Benutzereingabe eine Spracheinabe ist, die Spracheingabe in eine Texteingabe umgewandelt wird, und die erste Eingabe abhängig von der Texteingabe erzeugt wird.

10. Computerimplementiertes Verfahren nach einem der vorhergehenden Ansprüche, wobei das zentrale CIS (11) basierend auf Trainingsdaten trainiert ist oder trainiert wird, welche
- vorgegebene klinische Richtlinien und/oder Untersuchungsprotokolle und/oder Interventionsprotokolle für die medizintechnische Anlage (1) und/oder wissenschaftliche Veröffentlichungen betreffend mittels der medizintechnischen Anlage (1) durchführbare Untersuchungen und/oder Interventionen beinhalten; und/oder
- historische Benutzereingaben für das zentrale CIS (11) und/oder historische Konfigurationsdaten für die medizintechnische Anlage (1) beinhalten.

11. Computerimplementiertes Verfahren nach einem der vorhergehenden Ansprüche wobei das erste CIS (12) basierend auf weiteren Trainingsdaten trainiert ist oder trainiert wird, welche
- wenigstens ein Benutzerhandbuch und/oder wenigstens eine Bedienungsanleitung und/oder wenigstens eine Beschreibung für die erste Vorrichtung (2) beinhalten;
- vorgegebene klinische Richtlinien und/oder Untersuchungsprotokolle und/oder Interventionsprotokolle für die erste Vorrichtung (2) und/oder wissenschaftliche Veröffentlichungen betreffend mittels der ersten Vorrichtung (2) durchführbare Untersuchungen und/oder Interventionen beinhalten; und/oder
- historische Konfigurationsdaten und/oder Steuerungsdaten für die erste Vorrichtung (2) beinhalten.

12. Computerimplementiertes Verfahren nach einem der vorhergehenden Ansprüche, wobei
- das zentrale CIS (11) ein trainiertes künstliches neuronales Netzwerk beinhaltet, welches als Transformer-Netzwerk ausgestaltet ist; und/oder
- das erste CIS (12) ein weiteres trainiertes künstliches neuronales Netzwerk beinhaltet, welches als Transformer-Netzwerk ausgestaltet ist.

13. Datenverarbeitungsvorrichtung aufweisend wenigstens eine Recheneinheit, die dazu angepasst ist, ein computerimplementiertes Verfahren nach einem der vorhergehenden Ansprüche durchzuführen.

14. Medizintechnische Anlage (1) aufweisend eine Datenverarbeitungsvorrichtung nach Anspruch 13 sowie die erste Vorrichtung (2).

15. Computerprogrammprodukt aufweisend Befehle, die bei Ausführung durch eine Datenverarbeitungsvorrichtung die Datenverarbeitungsvorrichtung dazu veranlassen, ein computerimplementiertes Verfahren nach einem der Ansprüche 1 bis 12 durchzuführen.
